# EUROPÄISCHE PATENTSCHRIFT

(11) **EP 2 242 424 B1**
(45) Veröffentlichungstag und Bekanntmachung des Hinweises auf die Patenterteilung: **10.06.2020**
(21) Anmeldenummer: 09710235.4
(22) Anmeldetag: 11.02.2009
(51) Int. Cl.: A61B 5/15, A61B 5/151

(54) **VORRICHTUNG ZUR KÖRPERFLÜSSIGKEITSENTNAHME**
DEVICE FOR REMOVING BODILY FLUIDS
DISPOSITIF DESTINÉ AU PRÉLÈVEMENT DE LIQUIDES CORPORELS

(30) Priorität: 11.02.2008 EP 08151294
(43) Veröffentlichungstag der Anmeldung: 27.10.2010
(73) Patentinhaber: F. Hoffmann-La Roche AG, 4070 Basel (CH); Roche Diabetes Care GmbH, 68305 Mannheim (DE)
(72) Erfinder: LIST, Hans, 64754 Hesseneck-kailbach (DE); RÖDEL, Wolfgang, 69123 Heidelberg (DE); HÖRAUF, Christian, 68723 Oftersheim (DE)
(74) Vertreter: Pfiz, Thomas
(86) Internationale Anmeldenummer: PCT/EP2009/051581
(87) Internationale Veröffentlichungsnummer: WO 2009/101112

(56) Entgegenhaltungen:
- EP-A- 1 709 906
- WO-A-2005/084546
- WO-A2-2006/092309
- US-A1- 2007 064 516
- US-B1- 6 375 626

## Beschreibung

Die Erfindung betrifft eine Vorrichtung zur Körperflüssigkeitsentnahme mit einem in die Haut eines Körperteils einstechbaren Stechelement, das eine (insbesondere als Kapillare wirksame) Aufnahmestruktur für beim Hauteinstich gewonnene Körperflüssigkeit aufweist, und einem Stechantrieb für eine Vorwärts- und Rückziehbewegung des Stechelements, wobei die Dauer der Rückziehbewegung (vorzugsweise um ein Vielfaches) länger als die Dauer der Vorwärtsbewegung ist. Ein nicht unter die Ansprüche fallendes Beispiel betrifft weiter ein entsprechendes Verfahren zur Entnahme von Körperflüssigkeit.

Für Blutzuckertests wurde bereits vorgeschlagen, eine automatische Probenentnahme aus der Haut durch einen Einstich eines Stechelements dadurch zu bewerkstelligen, das die Rückziehbewegung deutlich langsamer erfolgt als die Vorwärtsbewegung, so dass eine für den Nachweis ausreichende Probenmenge zuverlässig gewonnen werden kann. Die Position des Übergangs von schneller zu langsamer Bewegung sollte dabei nur so tief im Gewebe liegen, das eine in das Stechelement eingearbeitete Aufnahmestruktur noch sicheren Kontakt zur austretenden Flüssigkeit bekommt. In der WO 2007/073870 ist ein Stechsystem beschrieben, mit welchem die Übergangsposition trotz variabler Stechtiefe mit allerdings erheblichem technischem Aufwand konstant gehalten werden kann. In EP 1709906 A1, US 2007/064516 A1 oder WO 2006/092309 A2 werden verschiedene Vorrichtungen zur Entnahme von Körperflüssigkeiten mit unterschiedlichen Stechprofilen beschrieben. In US6375626 B1 und WO 2005/084546 A2 werden Stechvorrichtungen zur Sammlung von Körperflüssigkeiten mit Sammelbehältern und Testelementen offenbart.

Ausgehend hiervon liegt der Erfindung die Aufgabe zugrunde, die im Stand der Technik vorgeschlagenen Systeme weiter zu verbessern und mit begrenztem baulichen Aufwand eine zuverlässige Probengewinnung zu gewährleisten und dabei auch die Schmerzhaftigkeit der Prozedur zu reduzieren.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, den Sammelbeginn auf eine Zwischenposition unter der Haut zu legen, die mit sehr hoher Geschwindigkeit angefahren werden kann, und die um eine feste Rückziehstrecke hinter der gewählten tiefsten Einstichposition liegt. Dabei ist es vorgesehen, das Stechelement in einer ersten Rückziehphase der Rückziehbewegung mit einer maximalen Rückziehgeschwindigkeit von mehr als 0,02 m/s zu bewegen. Durch diese Maßnahme kann die maximale Stechtiefe entsprechend den individuellen Hauteigenschaften so gewählt werden, dass genügend Blutkapillaren durch den Stich geöffnet werden, während diese besonders schmerzintensive Phase durch die schnelle erste Rückbewegung auf ein Minimum reduziert wird. In der nachfolgenden zweiten Rückziehphase findet dann erst der Sammelvorgang statt, der dadurch bestimmt ist, dass Körperflüssigkeit in die Aufnahmestruktur hineinfließt. Hierzu sollte der Hauteinstich hinreichend lange dauern, um die erforderliche Probenmenge aufnehmen zu können. Überraschend hat sich aber gezeigt, dass eine zu langsame Bewegung des Stechelements die Blutaufnahme behindert. Weiterhin sollte die Sammelphase innerhalb eines definierten Zeitintervalls abgeschlossen sein, um auch den Randbedingungen an die Einstechtiefe zu genügen. Dementsprechend wird erfindungsgemäß vorgeschlagen, dass der Stechantrieb dazu ausgebildet ist, das Stechelement während einer an die erste Rückziehphase anschließenden zweiten Rückziehphase zum Sammeln von Körperflüssigkeit in der Aufnahmestruktur so aus der Haut zurückzuziehen, dass die Rückziehgeschwindigkeit zwischen 0,6 und 2 mm/s beträgt und/oder die Sammeldauer im Bereich zwischen 0,3 und 0,8 s liegt. Gemäß einer bevorzugten Ausgestaltung wird das Stechelement während der ersten Rückziehphase um eine definierte erste Teilstrecke vorzugsweise von bis zu 0,5 mm aus der tiefsten Einstechposition in eine unter der Hautoberfläche liegende Zwischenposition zurückgezogen wird. Die Hautoberfläche kann dabei durch eine entsprechende Referenzposition beispielsweise über eine Positionierung für das Körperteil oder einen Hautdetektor oder eine vorgegebene Stechtiefe vorrichtungsseitig bestimmt sein. Weiterhin kann durch den konstanten ersten Rückzugweg auf eine technisch aufwendige Bewegungssteuerung verzichtet werden, wobei ein harmonischer Bewegungsablauf im Bereich der Richtungsumkehr der Nadel ohne schwingungskritische Anschlagstrukturen möglich wird.

Vorteilhafterweise erfolgt die Rückbewegung des Stechelements derart, dass die Aufnahmestruktur innerhalb der Sammeldauer mit Körperflüssigkeit zumindest überwiegend befüllbar ist, während das Stechelement noch in die Haut hineinragt. Hierbei ist zu bemerken, dass erst nach der ersten raschen Rückziehphase eine nennenswerte Aufnahme der in der Stichwunde freiwerdenden Körperflüssigkeit erfolgt.

Besonders günstig ist es, wenn die Dauer der Vorwärtsbewegung und der ersten Rückziehphase zwischen 0,3 bis 3 ms, vorzugsweise 0,3 bis 0,7 ms beträgt, so dass für den anfänglichen Stechvorgang ein harmonischer Bewegungsablauf ermöglicht wird.

Vorteilhafterweise sollte die Sammeldauer zur Aufnahme von Körperflüssigkeit in der Aufnahmestruktur zwischen 0,4 und 0,5 s betragen. In diesem Zusammenhang ist es auch besonders günstig, wenn die mittelere Rückziehgeschwindigkeit des Stechelements während der zweiten Rückziehphase im Bereich von 1 bis 1,5 mm/s liegt.

Für ein benutzerbezogenes Sammelprofil sollte die maximale Einstichtiefe zwischen 1 und 2,5 mm einstellbar sein.

Vorteilhafterweise ist der Geschwindigkeitsverlauf bei der Rückbewegung des Stechelements nach Maßgabe einer veränderlichen Stechtiefe so angepasst, dass das Stechelement für eine vorbestimmte Verweilzeit in der Haut eingestochen bleibt. Auf diese Weise ist eine individuelle Stechtiefenanpassung möglich, ohne dass die Zyklusdauern sich ständig ändern.

Für eine einfachere Bewegungssteuerung kann es vorteilhaft sein, wenn der Geschwindigkeitsverlauf bei der Rückbewegung des Stechelements unabhängig von der Stechtiefe vorgegeben ist.

Eine weitere Verbesserung hinsichtlich der Probengewinnung wird dadurch erzielt, dass die Geschwindigkeit des Stechelements in der zweiten Rückziehphase im Wesentlichen konstant ist.

Für eine vereinfachte Handhabung in einem integrierten System ist ein mit Körperflüssigkeit aus der Aufnahmestruktur beaufschlagbares Testelement vorgesehen.

Um eine nachteilige Probenveränderung weitgehend auszuschließen, sollte die Gesamtdauer vom Beginn der Rückziehbewegung des Stechelements bis zur Beaufschlagung des Testelements mit der Körperflüssigkeit weniger als 5 s, vorzugsweise weniger als 1 bis 2 s betragen.

Ein weiterer ergänzender oder alternativer Erfindungsaspekt liegt darin, dass ein für den Nachweis eines Analyten in der Körperflüssigkeit ausgebildetes Testelement so angeordnet ist, dass die Transferzeit zur Übertragung der Körperflüssigkeit aus der Aufnahmestruktur auf das Testelement (20) weniger als 1,5 s, vorzugsweise weniger als 1 s und bevorzugt weniger als 0,5 s beträgt. Überraschend hat sich herausgestellt, dass die Einhaltung dieses Zeitfensters besonders wichtig für die Testqualität ist. Das Testelement kann direkt auf dem Stechelement angeordnet sein und gegebenenfalls über eine Fließstrecke fluidisch mit der Aufnahmestruktur verbunden sein. Möglich ist es auch, das Testelement separat, insbesondere körperlich getrennt von der Aufnahmestruktur anzuordnen und die Flüssigkeit durch eine geeignete Aktuation beispielsweise unter Strukturverformung zu übertragen. Beispielhaft wird hier auf die WO 2005/084530 und WO 2007/025713 Bezug genommen.

Vorteilhafterweise besitzt das Stechelement ein beim Hauteinstich schmerzarm in die Haut eindringendes scharfes Stechorgan, insbesondere eine einzelne Nadelspitze.

Um mögliche Hauteindellungen beim Einstich zu berücksichtigen, kann die Position der Hautoberfläche durch einen Hautdetektor erfasst und/oder mittels einer Positioniereinheit für das Körperteil festgelegt werden.

Die Probengewinnung wird dadurch wesentlich vereinfacht, dass in das Stechelement eine zumindest an einem distalen Endabschnitt beim Hauteinstich in Kontakt mit der Körperflüssigkeit bringbare, insbesondere rinnen- oder schlitzförmige Kanalstruktur eingebracht ist.

Ein weiterer vorteilhafter Erfindungsaspekt liegt darin, dass der Antriebsmechanismus die Vorwärtsbewegung und Rückziehbewegung während einer ersten Rückziehphase des Stechelements steuert, während der Antriebsmotor das Stechelement in einer zweiten Rückziehphase der Rückziehbewegung aus der Haut zurückzieht. Auf diese Weise kann ein günstiges Bewegungsprofil mit einfachen technischen Mitteln realisiert werden. Dies ist besonders wichtig für in großer Stückzahl benötigte Testgeräte. Dabei kann der Antriebsmechanismus effektiv auf die schnelle Bewegung konzipiert werden, und der für die langsame Restbewegung vorgesehene Motor kann kompakt und energiesparend ausgelegt werden.

Vorteilhafterweise versorgt der Antriebsmotor den Antriebsmechanismus zur selbsttätigen Bewegungssteuerung mit mechanischer Energie.

Eine weitere Verbesserung wird dadurch erzielt, dass der Antriebsmotor den Antriebsmechanismus zusammen mit dem Stechelement als Verbund in der zweiten Rückziehphase zurückzieht.

Eine baulich besonders vorteilhafte Ausgestaltung sieht vor, dass der Antriebsmechanismus eine mittels einer Feder angetriebene Kurvensteuerung aufweist.

Ein nicht unter die Ansprüche fallendes Beispiel ist auch ein Verfahren zur Körperflüssigkeitsentnahme bei welchem durch einen Stechantrieb eine Vorwärts- und Rückziehbewegung eines Stechelements so gesteuert wird, dass das Stechelement in einer ersten Rückziehphase der Rückziehbewegung mit einer maximalen Rückziehgeschwindigkeit von mehr als 0,02 m/s zurück bewegt wird, und bei welchem das Stechelement während einer an die erste Rückziehphase anschließenden zweiten Rückziehphase zum Sammeln von Körperflüssigkeit in der Aufnahmestruktur so aus der Haut zurückgezogen wird, dass die Sammeldauer im Bereich zwischen 0,2 und 0,8 s liegt und/oder die Rückziehgeschwindigkeit zwischen 0,8 und 1,5 mm/s beträgt.

Die Erfindung wird definiert durch eine Vorrichtung nach Anspruch 1.

Im Folgenden wird die Erfindung anhand eines in der Zeichnung schematisch dargestellten Ausführungsbeispiels näher erläutert. Es zeigen:
- Fig. 1: eine Vorrichtung für Blutzuckertests mit einem mehrstufigen Stechantrieb in vereinfachter Schnittdarstellung;
- Fig. 2: ein Stechprofil beim Einsatz der Vorrichtung nach Fig. 1.

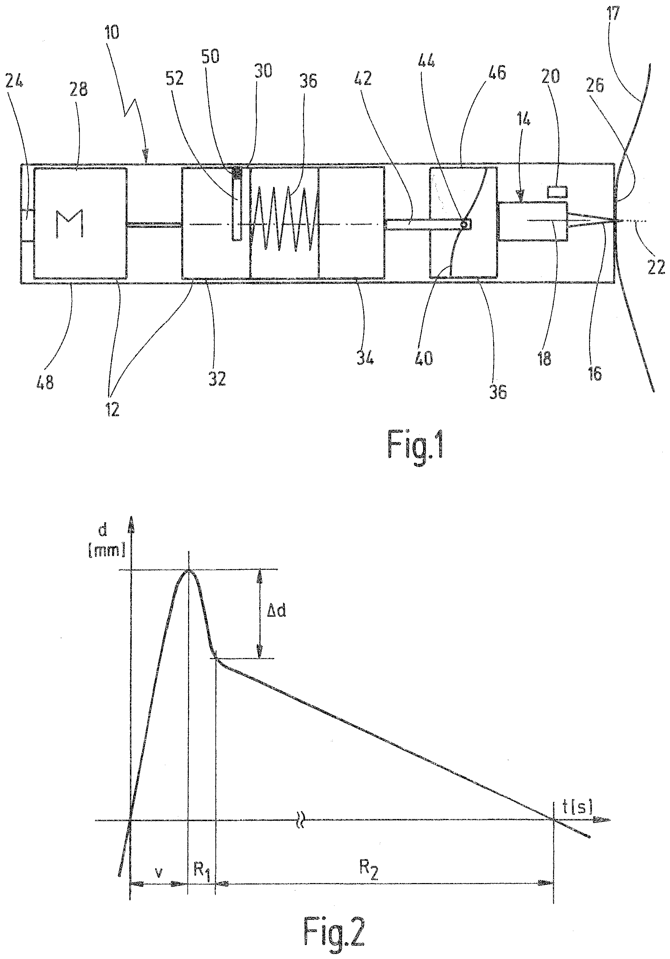

Die in Fig. 1 dargestellte Vorrichtung dient der Selbstentnahme einer Blutprobe durch einen Benutzer für Analysezwecke, insbesondere zur Blutzuckerkontrolle. Die Vorrichtung umfasst ein Handgerät 10 mit Stechantrieb 12 zur automatischen Handhabung eines als Einmalartikel zur Blutentnahme eingesetzten Stechelements 14.

Das Stechelement 14 ist als so genannter "Microsampler" zur Gewinnung einer kleinen Blutmenge aus einem Körperteil 17, insbesondere einer Fingerkuppe vorgesehen. Es kann als monolithisches einstückiges Formteil aus dünnem Edelstahlblech bestehen und eine distal angeformte Spitze 16 als Stechorgan zur Erzeugung einer Einstichwunde aufweisen. Ein mit seinem distalen Endabschnitt in den Bereich der Spitze 16 sich erstreckender rillen- oder schlitzförmiger Kapillarkanal 18 ermöglich die Aufnahme von Körperflüssigkeit (Blut und/oder Gewebeflüssigkeit) aus der Einstichwunde. Zum Nachweis der in der Körperflüssigkeit enthaltenen Zielsubstanz (Glucose) kann ein mit einer Testchemie versehenes Testelement 20 eingesetzt werden, welches nach dem Hauteinstich durch Herstellung einer geeigneten Fließverbindung mit Körperflüssigkeit aus der Aufnahmestruktur 18 beaufschlagbar ist. Der Blutglucosenachweis speziell mittels berührungsloser optischer Methoden ist im Stand der Technik an sich bekannt und wird daher hier nicht näher erläutert.

Der Stechantrieb 12 ermöglicht eine kontrollierte Vorwärts- und Rückziehbewegung des Stechelements 14 entlang einer Stechachse 22, wobei die Stechtiefe zweckmäßig im Bereich zwischen 1 und 2,5 mm zur Anpassung an verschiedene Hauttypen mittels einer Einstelleinheit 24 durch den Benutzer wählbar ist. Gegebenenfalls kann die Position der Hautoberfläche mittel einer Positioniereinheit 26 für das Körperteil 17 vorbestimmt werden.

Für eine mehrphasige Bewegungssteuerung umfasst der Stechantrieb 12 einen elektrisch arbeitenden Antriebsmotor 28 und einen damit vorgespannten, rein mechanisch arbeitenden Antriebsmechanismus 30. Der Antriebsmechanismus 30 steuert die schnelle Vorwärtsbewegung und eine erste schnelle Phase der Rückbewegung, während der Antriebsmotor 28 das Stechelement 14 über den Antriebsmechanismus 30 in einer zweiten Rückziehphase langsam aus der Haut zurückzieht. Auf diese Weise kann der Sammelvorgang optimiert und besonders benutzerfreundlich gestaltet werden.

Der mechanische Antriebsmechanismus 30 weist einen Spannrotor 32 und einen Antriebsrotor 34 auf, wobei die Rotoren über eine unter Vorspannung stehende Torsionsfeder 36 miteinander verbunden sind. Des Weiteren umfasst der Antriebsmechanismus 30 einen Kurven- bzw. Kulissentrieb 38, welcher die Rotationsbewegung des Antriebsrotors 34 über eine Steuerkurve 40 in eine Translations- bzw. Stechbewegung des angekoppelten Stechelements 14 umsetzt. Zu diesem Zweck greift ein an dem Antriebsrotor 34 abstehender Steuerarm 42 an seinem freien Ende über einen Kurvenreiter 44 in die umlaufende Steuerkurve 40 ein. Bei einer Drehung des Antriebsrotors 34 wird entsprechend der Kurvensteigung ein Hub erzeugt, wobei der Kurventrieb 38 über eine Linearführung 46 in dem Gerätegehäuse 48 geführt ist.

Die Relativdrehung der beiden Rotoren 32, 34 kann durch Anschlagelemente 50, 52 gegenseitig begrenzt werden, um die Vorspannung der Feder 36 aufzunehmen und den Antriebsrotor 34 in einer gewünschten Drehwinkelstellung zu stoppen. In einer vorbereitenden Spannphase wird der Antriebsrotor 34 drehfest gegenüber dem Gehäuse 48 arretiert, so dass die Feder 36 über den Spannrotor 32 durch Drehung des Motors 28 gespannt werden kann, bis die Ausgangsstellung der Anschlagelemente 50, 52 hergestellt ist. Die Arretierung des Antriebsrotors 34 wird in einer gegebenen Winkelstellung des Spannrotors 32 durch einen nicht gezeigten Auslöser aufgehoben, so dass der Antriebsrotor 34 augenblicklich federgetrieben dreht, bis das antriebsrotorseitige Anschlagstück 50 am anderen Ende der spannrotorseitigen Anschlagnut 52 anschlägt. Auf diese Weise kann ein Winkelbereich der Steuerkurve 40 abgefahren werden, um die Vorwärtsbewegung und die erste Rückziehphase der Rückziehbewegung sehr schnell auszuführen. Weitere Einzelheiten eines geeigneten Antriebsmechanismus sind in der EP-A 1 669 028 beschrieben.

Wie erwähnt, ist der Antriebsmotor 28 abtriebsseitig mit dem Spannrotor 32 gekoppelt, um in einer vorbereitenden Spannphase den Mechanismus 30 mit mechanischer Energie zu versorgen. Eine weitere wesentliche Funktion des Antriebsmotors 28 besteht in der kontrollierten langsamen Rückbewegung des Stechelements 14 während der zweiten Rückziehphase. Die Anschlagelemente 50, 52 werden dabei durch die verbleibende Federspannung in der oben beschriebenen Endstellung gehalten. Dadurch kann der Antriebsmechanismus 30 als Einheit weitergedreht werden, um den verbleibenden Abschnitt der Steuerkurve 40 abzufahren, wobei das Stechelement 14 mit definierter Rückziehgeschwindigkeit zurückgezogen wird. In dieser Phase kann das noch unter der Haut 16 befindliche Stechorgan 16 über die Sammelstruktur 18 aus der partiell freiwerdenden Stichwunde hinreichend Blut aufnehmen. Anschließend wird das aufgenommene Blut in einem Transferschritt durch eine geeignete Aktuation in bevorzugt weniger als 0,5 s auf das Testelement 20 übertragen. Zu diesem Zweck ist das Testelement 20 hinreichend nahe an der Aufnahmestruktur angeordnet, so dass der Flüssigkeitstransport unter Berücksichtigung der erreichbaren Transportgeschwindigkeit in der gegebenen Zeit erfolgt.

Für eine möglichst erfolgreiche und schmerzarme Blutaufnahme ist das in Fig. 2 gezeigte Stechprofil besonders vorteilhaft. Unter dem Begriff "Stechprofil" ist hierbei der Zeitverlauf der Stechbewegung zu verstehen, wie er als Funktion der Stechtiefe über der Zeit dargestellt ist.

In der Phase v der Vorwärtsbewegung trifft das Stechelement 114 mit seiner Spitze 16 bei t=0 mit einer hohen Geschwindigkeit auf der Haut auf und dringt in einem Zug bis zur gewünschten Einstichtiefe d vor. Diese Tiefe muss individuell optimiert werden, um durch die Epidermis hindurch die Blutkapillaren enthaltende Dermis zu erreichen. Die Dauer der Vorwärtsbewegung beträgt bevorzugt zwischen 0,3 bis 0,7 ms.

Sodann wird die Spitze 16 in der ersten Rückziehphase R1 um eine vorbestimmte Strecke Δd von ca. 0,5 mm auf eine unter der Hautoberfläche liegende Zwischenposition zurückgezogen. Bevorzugt liegt diese Rückziehposition im Stratum Corneum der Epidermis. Dieser erste Rückzug R1 sollte so rasch wie möglich erfolgen, weil das durch die scharfe Richtungsumkehr zu Schwingungen angeregte Stechelement 14 möglichst nicht zu viele Schwingungsperioden in der durchbluteten und innervierten Dermis ausführen sollte. Die kurz nach der Bewegungsumkehr erreichte maximale Rückziehgeschwindigkeit sollte daher mehr als 0,02 m/s betragen. Entsprechend wird die Dauer der ersten Rückziehphase im Bereich zwischen 0,3 und 3 ms begrenzt. Wie oben ausgeführt, wird ein einheitlicher harmonischer Bewegungsablauf in den Phasen v und R1 mittels des Antriebsmechanismus 30 erreicht.

Am Ende der Phase R1 wird der Rückzug des Stechelements 14 deutlich verlangsamt, so dass der Sammelvorgang während der anschließenden zweiten Rückziehphase R2 erfolgen kann. Hierbei hat sich überraschend gezeigt, dass die Rückziehgeschwindigkeit einen Mindestwert nicht unterschreiten und noch hinreichend hoch sein sollte, damit das Hautgewebe bereitwillig Flüssigkeit freigibt. Andererseits muss die Sammelzeit genügend lang sein, damit die Aufnahmestruktur bzw. Kapillare 18 die Flüssigkeit aufnehmen kann, was unter Berücksichtigung von Produktionstoleranzen und Alterungseffekten bis zu 500 ms dauern kann. Zu berücksichtigen ist auch, dass eine zu lange Verweildauer des Stechelements im eingestochenen Zustand in der Haut vom Anwender als lästig empfunden wird. Um eine hinreichende Blutaufnahme in der Aufnahmestruktur 18 zu erreichen, sollte die Geschwindigkeit des Stechelements in der zweiten Rückziehphase im Wesentlichen konstant sein, wobei ein Wert zwischen 1 und 1,5 mm/s günstig ist. Ein solcher vergleichsweise langsamer Rückzug kann durch Beaufschlagung eines kompakten Antriebsmotors 28 mit einer mit einfachen Mitteln konstant gehaltenen Spannung energiesparend erreicht werden.

Das Stechprofil kann unabhängig von der Stechtiefe fest vorgegeben sein. Bei einem tieferen Einstich wird dann die in Fig. 2 gezeigte Kurve gleichsam unverändert nach oben verschoben. Alternativ kann es von Vorteil sein, den Geschwindigkeitsverlauf in Abhängigkeit einer wahlweise veränderten Stechtiefe so anzupassen, dass eine definierte Verweilzeit im eingestochenen Zustand erreicht wird.

## Patentansprüche

1. Vorrichtung zur Körperflüssigkeitsentnahme mit einem in die Haut eines Körperteils (17) einstechbaren Stechelement (14), das eine insbesondere als Kapillare wirksame Aufnahmestruktur (18) für beim Hauteinstich gewonnene Körperflüssigkeit aufweist, und einem Stechantrieb (12) für eine Vorwärts- und Rückziehbewegung des Stechelements (14), wobei die Dauer der Rückziehbewegung länger als die Dauer der Vorwärtsbewegung ist und das Stechelement (14) in einer ersten Rückziehphase (R1) der Rückziehbewegung mit einer maximalen Rückziehgeschwindigkeit von mehr als 0,02 m/s angetrieben ist, **dadurch gekennzeichnet, dass** der Stechantrieb (12) dazu ausgebildet ist, dass das Stechelement (14) während der ersten Rückziehphase (R1) unabhängig von der Stechtiefe um eine konstante erste Teilstrecke (Δd) aus der tiefsten Einstechposition in eine unter der Hautoberfläche liegende Zwischenposition zurückgezogen wird, und das Stechelement (14) während einer an die erste Rückziehphase anschließenden zweiten Rückziehphase (R2) zum Sammeln von Körperflüssigkeit in der Aufnahmestruktur (18) so aus der Haut zurückzuziehen, dass die Sammeldauer im Bereich zwischen 0,3 und 0,8 s liegt und die Rückziehgeschwindigkeit zwischen 0,6 und 2 mm/s beträgt.

2. Vorrichtung nach Anspruch 1, **dadurch gekennzeichnet, dass** die erste Teilstrecke bis zu 0,5 mm beträgt.

3. Vorrichtung nach Anspruch 1 oder 2, **dadurch gekennzeichnet, dass** die Aufnahmestruktur (18) innerhalb der Sammeldauer mit Körperflüssigkeit zumindest überwiegend füllbar ist, während das Stechelement (14) in die Haut hineinragt.

4. Vorrichtung nach einem der Ansprüche 1 bis 3, **dadurch gekennzeichnet, dass** die Dauer der Vorwärtsbewegung zwischen 0,3 und 3 ms, vorzugsweise 0,3 bis 0,7 ms beträgt.

5. Vorrichtung nach einem der Ansprüche 1 bis 4, **dadurch gekennzeichnet, dass** die Dauer der ersten Rückziehphase (R1) zwischen 0,3 und 3 ms liegt.

6. Vorrichtung nach einem der Ansprüche 1 bis 5, **dadurch gekennzeichnet, dass** die Sammeldauer zum Sammeln von Körperflüssigkeit in der Aufnahmestruktur (18) zwischen 0,4 und 0,5 s beträgt.

7. Vorrichtung nach einem der Ansprüche 1 bis 6, **dadurch gekennzeichnet, dass** die mittlere Rückziehgeschwindigkeit des Stechelements (14) während der zweiten Rückziehphase (R2) im Bereich von 1,0 bis 1,5 mm/s liegt.

8. Vorrichtung nach einem der Ansprüche 1 bis 7, **dadurch gekennzeichnet, dass** die maximale Einstichtiefe (d) zwischen 1 und 2,5 mm einstellbar ist.

9. Vorrichtung nach einem der Ansprüche 1 bis 8, **dadurch gekennzeichnet, dass** der Geschwindigkeitsverlauf bei der Rückbewegung des Stechelements (14) nach Maßgabe einer veränderlichen Stechtiefe so angepasst ist, dass das Stechelement (14) für eine vorbestimmte Verweilzeit in der Haut eingestochen bleibt.

10. Vorrichtung nach einem der Ansprüche 1 bis 8, **dadurch gekennzeichnet, dass** der Geschwindigkeitsverlauf bei der Rückbewegung des Stechelements (14) unabhängig von der Stechtiefe vorgegeben ist.

11. Vorrichtung nach einem der Ansprüche 1 bis 10, **dadurch gekennzeichnet, dass** die Geschwindigkeit des Stechelements (14) in der zweiten Rückziehphase (R2) im Wesentlichen konstant ist.

12. Vorrichtung nach einem der Ansprüche 1 bis 11, **gekennzeichnet durch** ein mit Körperflüssigkeit aus der Aufnahmestruktur (18) beaufschlagbares Testelement (20) zum Nachweis eines Analyten in der Körperflüssigkeit.

13. Vorrichtung nach Anspruch 12, **dadurch gekennzeichnet, dass** die Gesamtdauer vom Beginn der Rückziehbewegung des Stechelements (14) bis zur Beaufschlagung des Testelements (20) mit der Körperflüssigkeit weniger als 5 s, vorzugsweise weniger als 1 bis 2 s beträgt.

14. Vorrichtung nach einem der Ansprüche 1 bis 13, **dadurch gekennzeichnet, dass** das Stechelement (14) einen zumindest an einem distalen Endabschnitt beim Hauteinstich in Kontakt mit der Körperflüssigkeit bringbaren, insbesondere rinnen- oder schlitzförmigen Kanal als Aufnahmestruktur (18) aufweist.

## Claims

1. Device for withdrawing body fluid with a lancing element (14) that can puncture the skin of a body part (17) which has a receiving structure (18) for body fluid obtained from the skin puncture which in particular acts as a capillary and a lancing drive (12) for a forward and retracting movement of the lancing element (14), wherein the duration of the retracting movement is longer than the duration of the forward movement and the lancing element (14) is moved in a first retraction phase (R1) of the rectracting movement at a maximum retraction speed of more than 0.02 m/s, **characterized in that** the lancing drive (12) is designed to pull back the lancing element (14 during the first retraction phase (R1) by a constant first partial distance (Δd) from the deepest puncture position into an intermediate position situated under the skin surface independently of the lancing depth, and to retract the lancing element (14) from the skin during a second retraction phase (R2) for collecting body fluid into the receiving structure (18) which follows the first retraction phase in such a manner that the collecting period is in a range between 0.3 and 0.8 s and the retraction speed is between 0.6 and 2 mm/s.

2. Device according to claim 1, **characterized in that** the first partial distance is up to 0.5 mm.

3. Device according to claim 1 or 2, **characterized in that** the receiving structure (18) can at least be substantially filled with body fluid during the collecting period while the lancing element (14) projects into the skin.

4. Device according to one of the claims 1 to 3, **characterized in that** the duration of the forward movement is between 0.3 and 3 ms, preferably 0.3 to 0.7 ms.

5. Device according to one of the claims 1 to 4, **characterized in that** the duration of the first retraction phase (R1) is between 0.3 and 3 ms.

6. Device according to one of the claims 1 to 5, **characterized in that** the collection period for taking up body fluid into the receiving structure (18) is between 0.4 and 0.5 s.

7. Device according to one of the claims 1 to 6, **characterized in that** the mean retraction speed of the lancing element (14) during the second retraction phase (R2) is in a range of 1.0 to 1.5 mm/s.

8. Device according to one of the claims 1 to 7, **characterized in that** the maximum lancing depth (d) is adjustable between 1 and 2.5 mm.

9. Device according to one of the claims 1 to 8, **characterized in that** the speed time course during the return movement of the lancing element (14) is adapted in accordance with a variable lancing depth in such a manner that the lancing element (14) remains inserted into the skin for a predetermined dwell period.

10. Device according to one of the claims 1 to 8, **characterized in that** the speed time course during the return movement of the lancing element (14) is preset independently of the lancing depth.

11. Device according to one of the claims 1 to 10, **characterized in that** the speed of the lancing element (14) in the second retraction phase (R2) is essentially constant.

12. Device according to one of the claims 1 to 11, **characterized by** a test element (20) for detecting an analyte in the body fluid to which body fluid can be applied from the receiving structure (18).

13. Device according to claim 12, **characterized in that** the total duration from the start of the retraction movement of the lancing element (14) until loading the test element (20) with the body fluid is less than 5 s, preferably less than 1 to 2 s.

14. Device according to one of the claims 1 to 13, **characterized in that** the lancing element (14) has an in particular groove-shaped or slot-shaped channel as the receiving structure (18) that can be brought into contact with the body fluid at least at a distal end section during skin puncture.

## Revendications

1. Dispositif pour le prélèvement de liquides corporels présentant un élément de piqûre (14), pouvant être piqué dans la peau d'une partie corporelle (17), qui présente une structure de réception (18) en particulier active comme capillaire pour le liquide produit lors de la piqûre dans la peau et un dispositif d'entraînement de piqûre (12) pour un mouvement vers l'avant et de retrait de l'élément de piqûre (14), la durée du mouvement de retrait étant plus longue que la durée du mouvement vers l'avant et l'élément de piqûre (14) étant entraîné, dans une première phase de retrait (R1) du mouvement de retrait, à une vitesse maximale de retrait supérieure à 0,02 m/s, **caractérisé en ce que** le dispositif d'entraînement de piqûre (12) est conçu pour que l'élément de piqûre (14), pendant la première phase de retrait (R1), soit retiré, indépendamment de la profondeur de piqûre, d'une première zone partielle constante (Δd) à partir de la position de piqûre la plus profonde dans une position intermédiaire située sous la surface de la peau et pour retirer l'élément de piqûre (14), pendant une deuxième phase de retrait (R2) consécutive à la première phase de retrait, de la peau pour collecter le liquide corporel dans la structure de réception (18) de manière telle que la durée de collecte se situe dans la plage entre 0,3 et 0,8 s et la vitesse de retrait est située entre 0,6 et 2 mm/s.

2. Dispositif selon la revendication 1, **caractérisé en ce que** la première zone partielle est de jusqu'à 0,5 mm.

3. Dispositif selon la revendication 1 ou 2, **caractérisé en ce que** la structure de réception (18) peut être remplie au moins principalement par le liquide corporel pendant la durée de collecte, alors que l'élément de piqûre (14) pénètre dans la peau.

4. Dispositif selon l'une quelconque des revendications 1 à 3, **caractérisé en ce que** la durée du mouvement vers l'avant est situé entre 0,3 et 3 ms et est de préférence de 0,3 à 0,7 ms.

5. Dispositif selon l'une quelconque des revendications 1 à 4, **caractérisé en ce que** la durée de la première phase de retrait (R1) se situe entre 0,3 et 3 ms.

6. Dispositif selon l'une quelconque des revendications 1 à 5, **caractérisé en ce que** la durée de collecte pour la collecte du liquide corporel dans la structure de réception (18) se situe entre 0,4 et 0,5 s.

7. Dispositif selon l'une quelconque des revendications 1 à 6, **caractérisé en ce que** la vitesse moyenne de retrait de l'élément de piqûre (14) pendant la deuxième phase de retrait (R2) se situe dans la plage de 1,0 à 1,5 mm/s.

8. Dispositif selon l'une quelconque des revendications 1 à 7, **caractérisé en ce que** la profondeur de piqûre maximale (d) est réglable entre 1 et 2,5 mm.

9. Dispositif selon l'une quelconque des revendications 1 à 8, **caractérisé en ce que** l'allure de la vitesse lors du mouvement de retrait de l'élément de piqûre (14) est adaptée en fonction d'une profondeur de piqûre variable de manière telle que l'élément de piqûre (14) reste enfoncé dans la peau pour un temps de séjour prédéfini.

10. Dispositif selon l'une quelconque des revendications 1 à 8, **caractérisé en ce que** l'allure de la vitesse lors du mouvement de retrait de l'élément de piqûre (14) est prédéfinie indépendamment de la profondeur de piqûre.

11. Dispositif selon l'une quelconque des revendications 1 à 10, **caractérisé en ce que** la vitesse de l'élément de piqûre (14) est sensiblement constante dans la deuxième phase de retrait (R2).

12. Dispositif selon l'une quelconque des revendications 1 à 11, **caractérisé par** un élément de test (20) pouvant recevoir du liquide corporel à partir de la structure de réception (18) pour la détection d'un analyte dans le liquide corporel.

13. Dispositif selon la revendication 12, **caractérisé en ce que** la durée totale à partir du début du mouvement de retrait de l'élément de piqûre (14) jusqu'à la réception du liquide corporel par l'élément de test (20) est inférieure à 5 s, de préférence inférieure à 1 jusqu'à 2 s.

14. Dispositif selon l'une quelconque des revendications 1 à 13, **caractérisé en ce que** l'élément de piqûre (14) présente au moins en une section d'extrémité distale, un canal, en particulier en forme de goulotte ou de fente, pouvant être amené en contact avec le liquide corporel lors de la piqûre dans la peau comme structure de réception (18).
